Europäisches Patentamt

European Patent Office

Office européen des brevets

(19)

(11) Publication number: **0 199 205**
**B1**

## EUROPEAN PATENT SPECIFICATION

(12)

(45) Date of publication of the patent specification:
**30.08.89**

(51) Int. Cl.⁴: **G01N 33/52**, G01N 31/22

(21) Application number: **86104949.2**

(22) Date of filing: **10.04.86**

(54) Sealed reagent matrix.

(30) Priority: **22.04.85 US 725752**

(43) Date of publication of application:
**29.10.86 Bulletin 86/44**

(45) Publication of the grant of the patent:
**30.08.89 Bulletin 89/35**

(84) Designated Contracting States:
**DE FR GB IT SE**

(56) References cited:
**EP-A- 0 013 156**
**EP-A- 0 045 476**
**EP-A- 0 126 357**
**US-A- 3 723 064**
**US-A- 3 996 006**
**US-A- 4 482 583**

(73) Proprietor: **MILES INC., 1127 Myrtle Street, Elkhart Indiana 46514(US)**

(72) Inventor: **Siddiqi, Sultan M., 54289 Blair Court, Elkhart Indiana 46515(US)**
Inventor: **Stemm, Patrick, 437 Arcade Avenue, Elkhart Indiana 46514(US)**

(74) Representative: **Dänner, Klaus, Dr. et al, c/o Bayer AG Konzernverwaltung RP Patentabteilung, D-5090 Leverkusen 1 Bayerwerk(DE)**

ACTORUM AG

## Description

### FIELD OF THE INVENTION

The present invention relates to sealing the edges of reagent matrix material and, more particularly, to reagent matrix material which has had a sealing composition applied to its edges to prevent or minimize cross-contamination of reagents during use.

### BACKGROUND OF THE INVENTION

The art of analytical chemistry has been greatly advanced since biochemistry began emerging as a primary scientific frontier, requiring increasingly sophisticated analytical methods and tools to solve problems. Likewise the medical profession has lent impetus to the growth of analytical chemistry, with its desiderata of both high precision and speed in obtaining results.

To satisfy the needs of the medical profession as well as other expanding technologies, such as the brewing industry and chemical manufacturing, a myriad of analytical procedures, compositions and apparatus have evolved, including the so-called "dip-and-read" type reagent test device. Reagent test devices enjoy wide use in many analytical applications, especially in the chemical analysis of biological fluids, because of their relatively low cost, ease of usability, and speed in obtaining results. In medicine, for example, numerous physiological functions can be monitored merely by dipping a reagent strip test device into a sample of body fluid, such as urine or blood, and observing a detectable response, such as a change in color or a change in the amount of light reflected from or absorbed by the test device.

Many of the "dip-and-read" test devices for detecting body fluid components are capable of making quantitative or at least semiquantitative measurements. Thus, by measuring the response after a predetermined time, an analyst can obtain not only a positive indication of the presence of a particular constituent in a test sample, but also an estimate of how much of the constituent is present. Such test devices provide the physician with a facile diagnostic tool as well as the ability to gage the extent of disease or of bodily malfunction.

Illustrative of such test devices currently in use are products available from the Ames Division of Miles Laboratories, Inc. under the trademarks CLINISTIX, MULTISTIX, KETOSTIX, N-MULTISTIX, DIASTIX, DEXTROSTIX, and others. Test devices such as these usually comprise one or more carrier matrices, such as absorbent paper having incorporated therein a particular reagent or reactant system which manifests a detectable response, e.g., a color change, in the presence of a specific test sample component or constituent. Depending on the reactant system incorporated with a particular matrix, these test devices can detect the presence of glucose, ketone bodies, bilirubin, urobilinogen, occult blood, nitrite, and other substances. A specific change in the intensity of color observed within a specific time range after contacting the test device with a sample is indicative of the presence of a particular constituent and/or its concentration in the sample. Some of these test devices and their reagent systems are set forth in US-A 3,123,443; 3,212,855 and 3,814,668.

Thus, it is customary for reagent test devices to contain more than one reagent bearing carrier matrix, in which each reagent bearing carrier matrix is capable of detecting a particular constituent in a liquid sample. For example, a reagent test device could contain a reagent bearing carrier matrix responsive to glucose in urine and another matrix responsive to ketones, such as acetoacetate, which is spaced from, but adjacent to, the glucose responsive matrix. Such a product is marketed by the Ames division of Miles Laboratories, Inc. under the trademark KETO-DIASTIX. Another reagent test device marketed by the Ames Division of Miles Laboratories, Inc., N-MULTISTIX, contains eight adjacent reagent incorporated matrices providing analytical measurement of pH, protein, glucose, ketones, bilirubin, occult blood, nitrite, and urobilinogen.

Despite the obvious, time-proven advantages of such multiple reagent test devices as these, misuse can result in misinformation. These multiple analysis tools comprise complex chemical and catalytic systems, each reagent matrix containing an unique reactive system, responsive to its particular analyte. Thus, it is possible, if the reagent test device is misused, for chemicals to be transported by the liquid sample being analyzed from one carrier matrix on the reagent test device to another. Should this happen it is possible for reagents from one carrier matrix to interfere with those of the other so contacted causing unreliable results. Although it is common in the reagent test device industry to provide detailed instructions of how this problem can be minimized, i.e., directions for properly manipulating the reagent test devices by blotting excess fluid, nevertheless ignorance or disregard of these instructions could permit reagents from one matrix to run over onto an adjacent one. Cross-contamination can result in false results. It is the prevention of this "runover" problem that the present invention is primarily directed.

The elimination of runover has been long sought after and the present discovery, which is the cumulation of an extensive research effort, provides a very effective solution to this problem.

### DISCUSSION OF THE PATENT LITERATURE

The patent literature is replete with accounts of myriad attempts at curtailing runover, the great bulk of the emphasis being directed to two basic concepts: the absorbance of runover liquid by bibulous layers placed beneath the reagent-bearing layers of reagent test devices; and the use of hydrophobic barriers between the spaced matrices. The former has met with moderate success, whereas the latter approach has not.

Of the multilayer type reagent test devices, US-A 4,160,008 describes a test device in which the carrier matrices containing reagent formulations are provided with adsorbent underlayers which are separated therefrom by sample impervious barrier layers. Each matrix thus forms the upper layer of a laminate composite in which the barrier layer is disposed between the matrix and the adsorbent base layer, the composite being fixed to a suitable support such as a plastic substrate. When the test device is dipped into the liquid sample the portion of sample which would otherwise runover from one matrix to another is largely adsorbed into the underlayer of the latter through the exposed sides, the barrier layers of the composite preventing the adsorbed runover from reaching the upper reagent layers.

US-A 4,301,115 discloses and claims a test device comprising a base support member coated with a hydrophobic barrier layer to which a plurality of spaced apart reagent matrices are affixed. This approach virtually eliminates cross-contamination between adjacent reagent areas of multiple reagent test devices, but requires an extra step of applying hydrophobic material to the base support member of the reagent test device.

With respect to the development and use of barriers and/or barrier materials between reagent matrices, the patent literature is replete with teachings, which in theory, at least, would seem to overcome the runover problem.

US-A 3,418,083 discloses an indicator-impregnated adsorbent carrier matrix treated with wax, oil or similar "hydrophobic" agents. It is stated that when a sample of blood is placed on the resulting reagent test device, only colorless liquid components permeate it, the proteinaceous, colored blood components remain on the surface where they can be removed. Thus, it is taught that the liquid portion bearing the analysate permeates the reagent matrix pad and color interference is precluded.

Still another patent, US-A 3,001,915, describes an adsorbent paper reagent test device having spaced reagent-impregnated test areas for more than one sample component, each such area being separated from the other reagent-impregnated test area by a nonadsorbent barrier portion. The barrier is provided by impregnation of the paper strip with materials such as polystyrene, rosin, paraffin and various cellulose esters. The reagent strip is prepared, according to the reference, by impregnating a portion of the paper strip with a glucose sensitive reagent system. When dry, a solution of one or more of the barrier materials is applied to the paper adjacent a glucose sensitive portion. After further drying a protein sensitive reagent system is applied and the process is repeated with alternate applications of reagent and barrier solutions with drying steps inbetween.

Yet an earlier patent, US-A 2,129,754, describes the impregnation of filter paper with paraffin wax whereby specific areas are left unimpregnated and these areas are treated with indicator systems for a particular analyte.

In US-A 3 006 735 the concept of barrier material impregnated between reagent areas of a reagent test device is carried one step further by providing successive reagent areas responsive to different degrees of water hardness. Water repellent material, such as oils, waxes, silicones, and printer's varnish, is impregnated between these reagent test areas. Like the proceeding two patents this citation is restricted to paper or like bibulous material wherein reagent and barrier material alike are impregnated sequentially along the test device.

US-A 4 482 583 describes a process for sealing the edges of reagent ribbons with liquid paraffin, oil or silicone.

EP-A 45 476 describes a device for separation of blood which separation properties can be improved by applying a hydrophobic barrier onto a glass fiber matrix embodied in this device.

In EP-A 126 357, various materials, including waxes, are suggested for sealing the edges of reagent matrix material such that liquid present in the reagent matrix will be retained therein and prevented from running over into another reagent matrix area present on the same reagent test device.

US-A 3 011 874 and 3 127 281 teach the use of hydrophobic barrier materials impregnated in part of a reagent test device in order to separate one reagent area from another and thereby avoid contamination. Yet another patent which mentions the separation of indicator reagent sites by the use of nonadsorbent or hydrophobic materials is US-A 3 964 871.

Currently marketed reagent test device products, for the most part, contain reagent impregnated matrices affixed to hydrophobic organoplastic material. Thus, the multiple reagent test device known as N-MULTISTIX contain eight different reagent impregnated matrices mounted on a polystyrene film. Since polystyrene is hydrophobic, the reagent strip can be said to have hydrophobic interstices between adjacent matrices.

Despite lip service given by literature accounts to the elimination of runover, the fact remains that the problem continues to exist. The approaches disclosed in US-A 4,160,008 and 4,301,115 have come the closest to eliminating the runover problem.

Prior attempts using wax, oils, silicones, and the like materials, have not curtailed runover to a clinically significant extent; and what modest advances have been made are more than offset by serious drawbacks inherent to such attempts. For example, applying hydrophobic material only at reagent area interstices embodies technical problems, especially when compared with the current techniques for manufacturing dip-and-read reagent test devices. Besides the obvious extra steps required by interstitial application, there is the danger of some of the hydrophobic material overlapping the reagent area thereby interfering with the paramount purpose of the reagent test device. Moreover, none of the substances taught by the prior art provides a suitable surface for adhesion.

Even if the above shortcomings were not prohibitive enough, the prior art hydrophobic substrates lack a degree of hydrophobicity required to prevent runover. They do not provide a sufficient contact angle to achieve the required hydrophobicity, nor do they provide a suitable surface for binding either the adsorbent matrices or the 9reagents themselves, where they are coated directly on the substrate surface.

The present invention virtually eliminates cross-contamination between adjacent reagent areas of multiple reagent test device matrices. The results are truly incontrovertible and the success achieved in solving this problem compares favorably with the use of a hydrophobic barrier layer, as described in US-A 4,301,115. Moreover, the present invention does not require the presence of an additional layer applied to the substrate of reagent test devices. The present invention, involving the sealing of one or both facing edges of a reagent matrix area, can be accomplished quickly and accurately.

SUMMARY OF THE INVENTION

An object of the present invention is to seal one or both facing edges of each reagent matrix of a reagent strip test device containing multiple carrier matrices in a way which prevents or substantially eliminates runover problems.

Another object of the present invention is to substantially eliminate runover problems by sealing one or both facing edges of each reagent matrix during the production of reagent test devices in a manner which does not interfere with the impregnated reagents in any individual carrier matrix.

Still another objection of the present invention is to provide an inexpensive and effective means of eliminating or materially reducing runover.

In accordance with the present invention, one or both facing edges of each reagent matrix are sealed with a specially formulated mixture of wax and adhesive. The specially formulated mixture consists of a high viscosity hot-melt adhesive combined with a wax which can be heated to a temperature of between approximately 90°C and 150°C and applied by a conventional hot-melt machine or other suitable means at one or more edges of reagent ribbon matrix material. Normally, the mixture is applied after the matrix material has been laminated onto a suitable substrate.

DESCRIPTION OF THE PREFERRED EMBODIMENTS

In accordance with the present invention, reagent matrix material is sealed with a specially formulated sealing mixture applied to one or both facing edges of each reagent matrix material. By applying sealing material only to the edges of the reagent matrix, reagent in the impregnated matrix is not affected. Accordingly, upon drying, the sealing material effectively seals one or more opposing ends or edges of the reagent matrix material and does not interfere with the reaction which takes place when the resulting reagent test device is contacted with liquid, such as a body fluid or an industrial liquid, to be tested.

The sealing material utilized to seal the edges of the reagent matrix material is a specially formulated mixture of wax and a high viscosity hot-melt adhesive. Broadly, the ratio of wax to adhesive can be from 1:1 to 1:4. Preferably, however, the ratio of wax to adhesive is 1:3. In addition to water-repellency, the sealing material has the characteristic of reasonable viscosity at elevated temperatures such that it can be easily applied to the edges of reagent ribbon and then quickly dried at room temperature or a slightly elevated temperature and remain solid over the normal temperature range in which reagent test devices are used. Clearly, no interaction must occur between the sealing material and the reagent(s) present in the reagent ribbon.

Waxes which are especially useful for use in the sealing material formulations are thermoplastic, water repellent, smooth in texture, nontoxic, and have freedom from any objectionable odor or color. Major types of waxes which can be employed include natural waxes, such as animal wax, beeswax, spermaceti, lanolin, shellac wax; vegetable waxes, such as carnauba, candelilla, bayberry, sugar cane; mineral waxes, such as fossil or earth waxes, including ozocerite, ceresin, montan; and petroleum waxes, such as paraffin, microcrystalline, petrolatum; as well as synthetic waxes such as ethylenic polymers and polyolether-esters including Carbowax®, sorbitol and chlorinated napthalenes such as Halowax® and other hydrocarbon waxes. A preferred wax is the WW0404 wax from H. B. Fuller Company of Kalamazoo, Michigan, which has the following characteristics: Melting point (ASTM D127) 82°C ± 4%, hydrophobic, inert, bendable and not tacky when dry. The congeal point (ASTM D938) is 76°C ± 4%, viscosity (Brookfield Thermocal) is 17.5 mPas (cps) 93°C, and color (ASTM D1500) is 1.0 Saybolt.

The hot-melt adhesive is also a thermoplastic material which can be a polyolefin, an olefin copolymer, polyester or polyamide. Among the polyolefins, polyethylene and polypropylene are particularly applicable. Copolymers of ethylene and vinylacetate are also useful. Polyamide hot-melt adhesives can be used, but tend to be more expensive. Polyester adhesives are also fairly high in price, but have good solvent resistance. Advantageously, the adhesive employed must have a fast set time. A particularly preferred hot-melt adhesive is the Eastobond® hot-melt adhesive A-3 which is made by Eastman Kodak Company, Kingsport, Tennessee. This adhesive has a thermal conductivity at 33°C and $10^{-4}$·4.184 Joules/cm-sec. of 7.1; a heat of fusion of 13.4·4.184 Joules per gram and a specific heat at 25°C of 0.58·4.184 Joules/gram. The Eastobond adhesive has a viscosity at application temperature of approximately 2200 mPa·s (centipoises).

The combined wax/adhesive formulation ideally has a softening point in excess of 70°C and no component melting below 80°C. The formulation is capable of drying quickly, i.e., in about 10 seconds, under ambient conditions and is not tacky when cold. The resulting mixture is hydrophobic, inert, slittable, colorless, insoluble in water and adheres readily to both paper and polystyrene.

The reagent ribbon or matrix material can be formed from any suitable material. US-A 3,846,247 teaches the use of felt, porous ceramic material and woven or matted glass fibers. Additionally, US-A 3,552,928 teaches the use of wood, cloth, sponge material and argillaceous substances. The use of synthetic resin fleeces and glass fiber felts as carrier matrix is suggested in GB-A 1,369,139. GB-A 1,349,623, proposes the use of light permeable meshwork of thin filaments as a cover for an underlying paper matrix. Polyimide fibers are taught in FR-A 2,170,397. Notwithstanding these suggestions, however, the material predominantly used in the art as a carrier matrix and that which is especially useful in the present invention is bibulous paper, such as filter paper.

As indicated above, the reagent ribbon is normally impregnated with reagent material prior to the sealing of the edges of the reagent ribbon in accordance with the present invention. In fact, in a preferred embodiment the reagent ribbon is attached to a card of suitable substrate material such as Trycite® (polystyrene) using double faced adhesive tape such as Double Stick, available from the 3M Company, prior to the application of the sealing material to one or more edges of the reagent ribbon. Thus, following conventional techniques multiple reagent ribbons are applied to a substrate card and then a hot-melt machine, such as the Nordson hot-melt device, which is manufactured and sold by the Packaging & Assembly Division of Nordson Corporation, is used to apply the specially formulated mixture of wax and adhesive to one or both of the exposed edges of the reagent ribbon. While the specially formulated wax/adhesive mixture can be applied to both of the exposed edges of the reagent ribbons, it has been found that normally the wax/adhesive mixture does not have to be applied to both sides of each reagent ribbon provided that the wax/adhesive mixture is applied to the same side of each matrix such that at least one barrier is formed between each of the resulting matrices on a reagent test device. Following application of the hot-melt admixture, the card containing reagent ribbons adhesively bound thereto is then cut widthwise in conventional manner to form reagent test devices. These reagent test devices can measure, for example, 8 x 0.5 centimeters having squares of reagent laden carrier matrices 0.5 centimeter on a side at one end thereof; the other end serving as a handle for the reagent test device. Since at least one edge of the reagent matrix material on each matrix pad is sealed by the sealing material in accordance with the present invention, liquid runover problems and the problems created by runover are effectively eliminated or substantially reduced.

EXAMPLE

Using a Nordson hot-melt machine, a specially formulated mixture of wax and adhesive containing 25 percent of H. B. Fuller's WW04040 wax and 75 percent of Eastman Kodak's A-3 hot-melt adhesive was heated to 93°C and then applied using a 0.0635 centimeter orifice nozzle to the edge of a continuous reagent impregnated filter paper ribbon which had been attached by means of double backed adhesive to polystyrene substrate material.

By varying the composition of the reagents so as to have a reagent test device with alternating pads of reagents suitable for the analysis of urobilinogen and nitrite and by applying the wax/adhesive admixture to only one side of each of the matrices, it was found to be possible to achieve 95 percent elimination of runover between the urobilinogen and nitrite reagent areas. Traditionally, the reagents for determination of these two analytes create the worst runover problem when they are both on the same test device. Thus, utilizing the present procedure, it is possible to apply reagent areas for measuring urobilinogen and nitrite adjacent to each other on the same reagent test device and it is no longer necessary to separate these reagent areas on the reagent test device or utilize more elaborate and costly means for overcoming the runover problem between adjoining spaced carrier matrices.

In contrast to the application of wax alone along the edges of reagent matrix material, the test devices prepared in accordance with the present invention have improved adhesion which is important because of the necessity to bond or adhere to different types of materials, e.g., polystyrene and paper. Moreover, the softening point of the wax/adhesive admixture is substantially improved over that of the wax alone. This is important because of the melting problem which can occur during shipment of reagent test devices when the containers in which the reagent test devices are stores are subjected to temperatures of 66° centigrade or higher. A stability problem has occurred when waxes were used to seal reagent matrix material and the resulting test devices were subjected during shipment to relatively high temperatures which would cause the wax to soften or melt and then spread into areas which interfered with the use of the test devices. In extreme cases, the melting wax can even bind with other test devices in the shipping container. The specially formulated wax/adhesive admixture of the present invention overcomes these problems in addition to overcoming the runover problem between adjoining spaced carrier matrices.

From the foregoing, it will be seen that this invention is well adapted to attain all of the ends and objects hereinabove set forth, together with other advantages which are obvious and which are inherent to the system. The wax/adhesive admixture represents a marked improvement over the use of wax alone in terms of both ease of application and effectiveness. For example, the present invention has the advantages of convenience, simplicity, relative inexpensiveness, positiveness, effectiveness, durability, accuracy and directness of action. The wax/adhesive mixture adheres firmly at the point of application and does not spread or interfere with the reagents present in the matrices. The invention substantially overcomes problems associated with runover which have been a continuing and long felt problem with multiple reagent test devices. The invention provides a very effective, simple and inexpensive way of eliminating or materially reducing the runover problem. In addition, the present invention can effectively be utilized in conjunction with conventional techniques, or methods for forming reagent test devices. There is no extra layer which must be applied to reagent test devices in order to control the runover problem. Moreover, the present invention could, if desired, be used in conjunction with other techniques found useful to control the runover problem. Thus, the present invention could be utilized in conjunction with techniques in the prior art which rely on the use of hydrophobic barrier layers affixed to reagent test devices.

## Claims

1. A reagent test device comprising multiple reagent carrier matrices attached in spaced relationship to one side of the substrate, said reagent carrier matrices being impregnated with a water-repellent wax/high viscosity hot melt adhesive admixture in a ratio of from 1:1 to 1:4 sealing at least one facing edge on each carrier matrix to prevent liquid runover between adjoining spaced carrier matrices, said wax/adhesive admixture having a softening point greater than 70°C and a melting point greater than 80°C.

2. The test device of claim 1 in which the carrier matrix is filter paper.

3. The test device of claim 1 in which the substrate is polystyrene.

4. The test device of any of claims 1 to 3 in which the wax/adhesive admixture is in the ratio of 1:3.

5. A process of sealing at least one facing edge of each reagent matrix area on a reagent test device containing multiple carrier matrices to substantially eliminate runover between adjoining spaced carrier matrices, which comprises applying an admixture of a water repellent wax and a high viscosity hot-melt adhesive as a hot-melt along each edge of reagent carrier matrix material which is to be sealed, said wax/adhesive admixture having a softening point greater than 70°C and a melting point greater than 80°C.

6. The process of claim 6 in which the carrier matrices are filter paper.

7. The process of claim 6 in which the substrate is polystyrene.

8. The process of claim 6 in which the carrier matrix material is filter paper which is laminated to polystyrene substrate prior to the time of applying the wax/adhesive admixture.

## Patentansprüche

1. Reagens-Testeinrichtung aus Mehrfach-Reagensträgermatrizes, die auf Abstand an einer Seite des Substrates befestigt sind, wobei die Reagensträgermatrizes mit einer wasserabstossenden Wachs/hochviskosen Hot-melt-Kleber-Mischung in einem Verhältnis von 1:1 bis 1:4 imprägniert sind und wenigstens eine gegenüberliegende Kante auf jeder Trägermatrix zur Verhinderung eines Flüssigkeitsüberlaufes zwischen angrenzenden, auf Abstand stehenden Trägermatrizes dadurch versiegelt wird, wobei das Wachs/Klebstoff-Gemisch einen Erweichungspunkt von mehr als 70°C und einen Schmelzpunkt von mehr als 80°C hat.

2. Testeinrichtung gemäss Anspruch 1, in welcher die Trägermatrix Filterpapier ist.

3. Testeinrichtung gemäss Anspruch 1, in welcher das Substrat Polystyrol ist.

4. Testeinrichtung gemäss einem der Ansprüche 1 bis 3, in welcher die Wachs/Klebstoff-Mischung in einem Verhältnis von 1:3 vorliegt.

5. Verfahren zum Versiegeln von wenigstens einer gegenüberliegenden Kante von jeder Reagensmatrixfläche auf einer Testeinrichtung, die mehrere Trägermatrizes enthält, um dadurch ein überlaufen zwischen anliegenden, auf Abstand stehenden Trägermatrizes zu unterbinden, dadurch gekennzeichnet, dass man eine Mischung aus einem wasserabstossenden Wachs und einem hochviskosen Hot-melt-Kleber als Hot-melt längs der zu versiegelnden Kante des Reagensträgermatrixmaterials aufträgt, wobei das Wachs/Klebstoff-Gemisch einen Erweichungspunkt von mehr als 70°C und einen Schmelzpunkt von mehr als 80°C hat.

6. Verfahren gemäss Anspruch 5, bei dem die Trägermatrizes Filterpapier sind.

7. Verfahren gemäss Anspruch 5, in welchem das Substrat Polystyrol ist.

8. Verfahren gemäss Anspruch 5, in welchem das Trägermatrixmaterial Filterpapier ist, das vor dem Zeitpunkt, zu dem die Wachs/Klebstoff-Mischung aufgebracht wurde, auf Polystyrol laminiert wurde.

## Revendications

1. Dispositif analytique réactif comprenant un grand nombre de matrices de support de réactifs fixées à un côté du substrat de manière à être séparées les unes des autres, lesdites matrices de support de réactifs étant imprégnées d'un mélange cire hydrofuge/adhésif thermofusible de haute viscosité en un rapport de 1:1 à 1:4 assurant l'étanchéité d'au moins un bord antérieur de chaque matrice de support afin d'empêcher un débordement de liquide entre des matrices de support séparées adjacentes, ledit mélange cire/adhésif ayant un point de ramollissement supérieur à 70°C et un point de fusion supérieur à 80°C.

2. Dispositif analytique suivant la revendication 1, dans lequel la matrice de support est du papierfiltre.

3. Dispositif analytique suivant la revendication 1, dans lequel le substrat est du polystyrène.

4. Dispositif analytique suivant l'une quelconque des revendications 1 à 3, dans lequel le mélange cire/adhésif est présent en un rapport égal à 1:3.

5. Procédé pour assurer l'étanchéité d'au moins un bord antérieur de chaque zone de matrice de réactif sur un dispositif analytique réactif contenant un grand nombre de matrices de support pour supprimer pratiquement le débordement entre des matrices de support séparées adjacentes, qui consiste à appliquer un mélange d'une cire hydrofuge et d'un adhésif thermofusible de haute viscosité sous forme d'une colle thermofusible, le long de chaque bord de matrice de support de réactif dont l'étanchéité doit être assurée, ledit mélange cire/adhésif ayant un point de ramollissement supérieur à 70°C et un point de fusion supérieur à 80°C.

6. Procédé suivant la revendication 5, dans lequel les matrices de support sont constituées de papierfiltre.

7. Procédé suivant la revendication 6, dans lequel le substrat est du polystyrène.

8. Procédé suivant la revendication 6, dans lequel la matière de la matrice de support est du papierfiltre qui est stratifié à un substrat de polystyrène avant d'appliquer le mélange cire/adhésif.